(19) Europäisches Patentamt
European Patent Office
Office européen des brevets

(11) **EP 1 296 640 B1**

(12) **FASCICULE DE BREVET EUROPEEN**

(45) Date de publication et mention de la délivrance du brevet:
**03.06.2009 Bulletin 2009/23**

(51) Int Cl.:
***A61K 8/81*** *(2006.01)*     ***A61Q 5/00*** *(2006.01)*
***A61Q 19/00*** *(2006.01)*

(21) Numéro de dépôt: **01947510.2**

(22) Date de dépôt: **15.06.2001**

(86) Numéro de dépôt international:
**PCT/FR2001/001892**

(87) Numéro de publication internationale:
**WO 2001/097772 (27.12.2001 Gazette 2001/52)**

(54) **UTILISATION COMME EPAISSISSANTS EN COSMETIQUE DE COPOLYMERES NEUTRALISES COMPORTANT DES MOTIFS D'ACIDE FAIBLE ET DES MOTIFS D'ACIDE FORT**

VERWENDUNG ALS VERDICKUNGSMITTEL IN DER KOSMETIK, VON NEUTRALISIERTEN COPOLYMEREN MIT SCHWACHEN SÄURE- UND STARKEN SÄUREEINHEITEN

USE AS THICKENERS IN COSMETICS OF NEUTRALIZED COPOLYMERS COMPRISING WEAK ACID UNITS AND STRONG ACID UNITS

(84) Etats contractants désignés:
**AT BE CH CY DE DK ES FI FR GB GR IE IT LI LU MC NL PT SE TR**

(30) Priorité: **23.06.2000 FR 0008201**

(43) Date de publication de la demande:
**02.04.2003 Bulletin 2003/14**

(73) Titulaire: **SNF**
**F-42000 Saint-Etienne (FR)**

(72) Inventeurs:
• **VILLARD, Emmanuel,**
**c/o SNF SA**
**42163 Andrezieux Cédex (FR)**
• **BLONDEL, Frédéric,**
**c/o SNF SA**
**42163 Andrezieux Cédex (FR)**

(74) Mandataire: **Richebourg, Michel François et al**
**Cabinet Michel Richebourg,**
**"Le Clos du Golf",**
**69, rue Saint-Simon**
**42000 Saint Etienne (FR)**

(56) Documents cités:
**EP-A- 0 321 650**     **EP-A- 0 341 660**
**EP-A- 0 341 662**     **EP-A- 0 503 853**
**EP-A- 1 046 390**     **EP-B- 0 161 038**
**WO-A-99/36445**     **US-A- 4 401 650**
**US-A- 4 859 458**     **US-A- 4 906 701**
**US-A- 5 185 395**

Remarques:
Le dossier contient des informations techniques présentées postérieurement au dépôt de la demande et ne figurant pas dans le présent fascicule.

EP 1 296 640 B1

**Description**

La présente invention concerne le secteur technique des compositions cosmétiques, dermatologiques, pharmaceutiques ou vétérinaires, et détergentes, comprenant au moins un polymère épaississant et / ou émulsionnant pour milieu aqueux.

On connaît dans l'art antérieur :

-   L'utilisation de polymères réticulés pour élever la viscosité de produits cosmétiques, pharmaceutiques ou techniques (FR 2,416,723)

-   L'utilisation d'une dispersion de polymère comme épaississant de pâte d'impression pour textile (EP 0,161,038)

-   Les compositions comprenant un polymère épaississant à base de monomères non ioniques tels que l'acrylamide, anioniques tels que l'acide acrylique ou cationiques, gonflant à l'eau. Les polymères sont obtenus sous forme d'émulsion inverse. Sont citées entre autres des applications comme les cosmétiques et surtout les impressions textiles ou sur moquette (GB 2,077,750)

-   Les polymères épaississants obtenus sous forme d'émulsion inverse à base d'acides forts (US 4,172,066)

-   Le brevet EP 0 503 853 qui concerne un épaississant à base d'AMPS et d'acrylamide.

-   L'utilisation d'un monomère possédant un acide fort spécifiquement pour épaissir des milieux acides (application pétrole) (GB 2,110,744)

-   Le procédé de préparation d'une composition sous forme de latex inverse d'un polyélectrolyte (homopolymère) anionique, branché ou réticulé, à base d'un monomère possédant une fonction acide fort (WO 99/42521).

Des polymères branchés ou réticulés à base d'acide acrylamido méthylpropane sulfonique et d'acide acryliane sont des épaississants et/ou émulsionnants de composition cosmétiques acides. (WO 99/36445).

On connaît encore le brevet EP 0 186 361.

Un problème technique extrêmement important dans l'industrie considérée consiste à apporter à la composition finale certaines propriétés intéressantes comme le toucher et la stabilité de la viscosité en présence d'électrolytes. Une forte demande existe, dont la solution n'a pas été apportée de manière satisfaisante par l'art antérieur.

Le problème technique correspondant est donc de rechercher des compositions présentant une capacité épaississante vis à vis de compositions aqueuses utilisables en cosmétique, dermatologie, dans le domaine vétérinaire ou pharmaceutique, et domaines analogues de produits de soins corporels, et notamment améliorant le toucher et la stabilité.

Plus particulièrement, on recherche des compositions épaississantes aussi bien à pH acide qu'à pH basique, et notamment celles efficaces à pH très acide, notamment pH 1 - 3 environ, ce qui correspond à un problème technique particulier, qui n'est pas correctement résolu par les produits de l'art antérieur, malgré une demande importante de l'industrie.

On entend, dans toute la présente demande par « polymère épaississant et/ou émulsionnant », un copolymère obtenu par polymérisation sous forme d'émulsion inverse tel que décrit dans le brevet EP 0,161,038.

Cependant, il reste encore à améliorer la stabilité de la composition en présence d'électrolytes. En effet, la viscosité (et donc la consistance) du produit final a tendance à chuter fortement au contact des électrolytes, par exemple de la peau. Il en résulte des inconvénients de confort d'utilisation et de performances.

Un problème technique encore plus fin et extrêmement important dans l'industrie considérée consiste donc à apporter à la composition finale certaines propriétés intéressantes comme la stabilité aux électrolytes. Une forte demande existe, dont la solution n'a pas été apportée de manière satisfaisante par l'art antérieur. Ainsi lorsque des caractéristiques dites « de toucher très frais » sont décrites, elles sont en fait la résultante directe d'une mauvaise tenue de la viscosité du polymère en présence d'électrolytes.

L'invention concerne une famille de polymères épaississants et/ou émulsionnants du type de celle décrite dans le brevet EP 0 161 038 précité, qui permet d'épaissir des compositions cosmétiques, dermatologiques, pharmaceutiques ou vétérinaires aussi bien à pH acide qu'à pH basique tout en apportant à la composition des caractéristiques cosmétiques optimales et jamais obtenues telles que le toucher et la stabilité de la viscosité en présence d'électrolytes. L'utilisation de ce type de polymères peut également être envisagée pour épaissir tout type de milieux aqueux, comme par exemple en détergence.

La présente invention concerne l'utilisation d'une sélection particulière de cette famille de copolymères comme agents épaississants et / ou émulsionnants ( en totalité ou en partie) de compositions cosmétiques, dermatologiques, pharmaceutiques, à usage humain ou vétérinaire, ou détergentes.

La composition obtenue présente de manière surprenante des caractéristiques cosmétiques optimales avec en particulier une très grande stabilité en présence d'électrolytes dans une gamme très large de pH (1-13) grâce à un choix approprié des conditions de polymérisation du polymère épaississant et/ou émulsionnant (taux de neutralisation, pourcentage de polymères solubles, rapport molaire acide fort/acide faible, concentration en matière active) conduisant à la fraction de polymères solubles précitée.

L'invention concerne donc l'utilisation comme épaississant et / ou émulsionnant pour milieux aqueux (en totalité ou en partie) de compositions cosmétiques, dermatologiques, pharmaceutiques, à usage humain ou vétérinaire, ou détergentes, d'au moins un polymère obtenu par polymérisation de:

- 5 à 95% molaire d'au moins un monomère possédant une fonction acide faible, et de

- 5 à 95% molaire d'au moins un monomère possédant une fonction acide fort et

- **caractérisé en ce qu**'il présente une fraction de polymères hydrosolubles comprise entre 5 et 50% en poids (par rapport au polymère total ), de préférence entre 8 et 35%, la méthode de détermination de la fraction hydrosoluble étant la suivante :

  - la méthode est basée sur la séparation des microgels de polymère réticulé d'avec une solution de polymère, par centrifugation, et la teneur en polymère est déterminée avant et après par titration de colloides, sur la base de la précipitation stoechiométrique de particules colloidales chargées par titration par un polymère de charge opposée, en utilisant un indicateur visuel,

  et en ce que, au momemt de la polymérisation, la neutralisation de la globalité des monomères possédant une fonction acide est partielle et comprise entre 5 et 95%.

Sans vouloir être liée par une quelconque théorie, la Demanderesse considère que cette caractéristique extrêmement importante au plan industriel et commercial réside de la proportion élevée de polymères hydrosolubles.

Les produits commerciaux les plus proches, qui déclarent « un toucher frais », donc une médiocre résistance aux électrolytes de la peau, ne dépassent pas 2 à 3 % de polymères hydrosolubles. Il semble donc exister un seuil de propriétés autour de 5% (cf. essai selon l'invention à 6 %). (les essais selon l'invention sont des moyennes de trois essais, afin de réduire la marge d'erreur au maximum).

On note par ailleurs que les polymères de la présente demande, ne comportent pas de monomères neutres, ce qui est probablement un facteur important dans l'obtention des avantages précités.

Selon un mode de réalisation absolument préféré, les polymères sont obtenus de plus en présence d'un agent réticulant ou ramifiant et éventuellement d'un agent de transfert.

Selon un mode de réalisation préféré, la polymérisation est effectuée en émulsion inverse eau dans huile.

Selon un mode de réalisation préféré, le taux de réticulation (ou ramification selon les cas) est de 50 à 3000 ppm (en considérant le méthylènebisacrylamide ou MBA) par rapport au polymère ou une réticulation équivalente avec un agent réticulant d'efficacité différente, selon des paramètres connus de l'homme de métier.

Dans les mêmes conditions, le polymère qui serait obtenu en l'absence d'agent de réticulation aurait une viscosité intrinsèque (I.V.) de 3 à 25 dl/g.

Selon un autre mode de réalisation, la concentration de polymérisation est de 15 à 55 % en poids.

Selon encore un autre mode de réalisation, la polymérisation est menée éventuellement en présence d'un agent de transfert.

L'homme de métier saura apprécier à partir de ses connaissances propres le degré d'agent de transfert et d'agent réticulant ou ramifiant à utiliser pour obtenir un polymère ramifié ou réticulé. L'homme de métier connaît également la définition chimique de ces deux catégories de polymères, de nombreux ouvrages et brevets antérieurs traitant de tels polymères. On indiquera simplement dans la présente demande « réticulé ou ramifié » ou « réticulé » et vocables analogues, pour rappeler ce qui précède.

Il est de plus essentiel qu'au moment de la polymérisation la neutralisation de la globalité des monomères possédant une fonction acide soit partielle et comprise entre 5 et 95%. Le pH de polymérisation varie en fonction du taux de neutralisation. Il est ainsi possible selon l'invention de travailler à un pH où les problèmes de corrosion des équipements industriels sont réduits très nettement, au point que des équipements spéciaux ne sont pas nécessaires. On travaillera notamment à pH > 4 en polymérisation, de préférence 4,2 - 4,5, en notant que, au dessous de pH 4 (et bien que la différence de pH en valeur absolue puisse sembler très faible), des équipements spéciaux anti-corrosion sont nécessaires. Ainsi la possibilité (non limitative) offerte par l'invention de travailler à des pH de polymérisation de légèrement plus de 4 représente un autre seuil technique intéressant et surprenant.

Grâce à ce choix approprié des conditions de polymérisation du polymère épaississant et/ou émulsionnant (taux de neutralisation, concentration en matière active, rapport molaire acide fort/acide faible), la composition obtenue présente de manière surprenante des caractéristiques cosmétiques optimales telles que le toucher et la stabilité dans une gamme très large de pH (1-13), tout en conservant un très fort et très efficace pouvoir épaississant, même à pH très acide, notamment même à pH 1 - 3 environ.

Une optimisation des conditions de polymérisation sera accessible à l'homme de métier à la lecture de la présente description et selon ses connaissances personnelles, ou à l'aide d'essais simples de routine.

Il est, de plus, également possible de concentrer ou d'isoler le polymère par toutes les techniques connues.

Il existe en particulier de nombreux procédés d'obtention de poudre à partir d'émulsions de polymères solubles ou gonflant dans l'eau qui consistent à isoler la matière active des autres constituants de l'émulsion. De manière non limitative, il est possible de citer les techniques telles que :

- La précipitation dans un milieu non solvant tel que l'acétone, le méthanol et autres solvants polaires. Une simple filtration permet alors d'isoler la particule de polymère.

- La distillation azéotropique en présence d'agent agglomérant et de polymère stabilisant qui permet de conduire à des agglomérats que l'on isole facilement par filtration avant de procéder au séchage de la particule.

- Le « spray-drying » ou séchage par atomisation ou pulvérisation qui consiste à créer un nuage de fines gouttelettes d'émulsion dans un courant d'air chaud, pendant une durée contrôlée.

Selon un mode de réalisation préféré, le copolymère est obtenu à partir de :

- 10 à 80% molaire d'au moins un monomère possédant une fonction acide faible

- et de 20 à 90% molaire d'au moins un monomère possédant une fonction acide fort.

Selon un mode de réalisation particulier, le taux de neutralisation (au moment de la polymérisation) de l'ensemble des acides insaturés est de préférence de 10 à 80%.

De manière tout à fait préférée, on incorpore un agent de réticulation ou de ramification et éventuellement un agent de transfert.

Selon encore un mode de réalisation particulier, le taux de réticulation est situé de préférence entre 100 et 800 ppm (en considérant le MBA) par rapport au polymère ou une réticulation équivalente avec un agent réticulant d'efficacité différente.

En l'absence d'agent de réticulation, la viscosité intrinsèque I.V. du polymère obtenu est de préférence 4 à 20 dl/g

La concentration de polymérisation est de préférence de 25 à 45% de matière active en poids.

Le taux global de neutralisation des monomères possédant une fonction acide est compris entre 20 et 90% à la polymérisation

Lors de la polymérisation sous forme d'émulsion eau dans huile, la phase continue utilisée peut être une huile ou un solvant d'origine minérale et/ou de synthèse et/ou d'origine végétale. De préférence, on utilisera un solvant ou une huile non minérale.

On trouvera ci dessous une liste non limitative des monomères de type acide fort :

- monomères présentant une fonction de type acide sulfonique, acide phosphonique, par exemple : l'acide 2-acrylamido-2-methylpropane sulfonique (AMPS).

On trouvera ci-dessous une liste non limitative des monomères présentant une fonction acide faible : l'acide acrylique, l'acide méthacrylique, l'acide itaconique, l'acide crotonique, l'acide maléique, l'acide fumarique, etc...

On trouvera ci dessous une liste non limitative des réticulants : méthylènebisacrylamide (MBA), l'ethylene glycol di-acrylate, le polyethylene glycol dimethacrylate, le diacrylamide, le cyanomethylacrylate, le vinyloxyethylacrylate ou methacrylate et le formaldehyde, le glyoxal, les composés de type glycidyléther comme l'éthylèneglycol diglycidyléther, ou des époxy ou tout autre moyen bien connu de l'homme de métier permettant la réticulation.

On trouvera ci dessous une liste non limitative des agents de transfert : alcool isopropylique, hypophosphite de sodium, mercaptoethanol etc...

On aura compris qu'il sera à la portée de l'homme de métier de sélectionner, dans les conditions et plages de mise en oeuvre ci-dessus, les conditions optimales telles que le polymère final présente une fraction de polymère hydrosoluble comprise entre 5 et 50 % en poids par rapport au polymère total (suivant une méthode de dosage du type de celle décrite p 8 du brevet EP 0 343 840), (A). L'homme de métier saura notamment apprécier à partir de ses connaissances propres le degré d'agent de transfert et d'agent réticulant ou ramifiant à utiliser pour obtenir un polymère final présentant une fraction de polymère hydrosoluble suffisante, et le taux de neutralisation à choisir pour obtenir un pH de polymérisation légèrement supérieur à 4 (s'il le souhaite).

L'homme de métier saura choisir la meilleure combinaison en fonction de ses connaissances propres et de la présente description, ainsi que des exemples qui vont suivre.

**Exemples de polymères :**

Chacun des polymères décrits ci-après a été obtenu par polymérisation sous forme d'émulsion inverse telle que décrite dans le brevet EP 0,161,038. Pour mieux pouvoir les comparer, les taux de réticulant et d'agent limiteur ont été gardés constants.

Il apparaît à la vue des résultats obtenus que le comportement (pouvoir épaississant et capacité de résistance aux électrolytes) est directement fonction des conditions de polymérisation et en particulier du taux de neutralisation et du rapport molaire acide fort/acide faible.

Le choix du polymère utilisé est ensuite fonction des caractéristiques requises pour la composition finale.

| | P1 | P2 | P3 | P4 | P5 | P6 | X |
|---|---|---|---|---|---|---|---|
| % molaire AMPS (acide fort) | 60 | 60 | 60 | 40 | 50 | 90 | 57 |
| % molaire Ac. Acrylique (acide faible) | 40 | 40 | 40 | 60 | 50 | 10 | 43 |
| % Neutralisation (total) | 70 | 50 | 3 6 | 55 | 60 | 70 | 57 |
| % Matière Active | 35 | 35 | 30 | 30 | 30 | 35 | 38 |
| % Polymère Hydrosoluble | 17 | 6 | 15 | 10 | 28 | 20 | 3 |
| QC1 (mesure de viscosité à pH=3) | 6000 | 16500 | 12000 | 3250 | 4500 | 12500 | 12000 |
| QC2 (chute de viscosité par ajout de sels) | 54 | 70 | 43 | 54 | 44 | 60 | 99,9 |

Il serait sans doute possible techniquement d'augmenter la proportion de polymères hydrosolubles au delà de 28 %, mais sans grand intérêt, car semble que l'on atteigne la limite du compromis autour de 25 - 30 - 35 % environ, en fonction des détails d'application.

Par contre, comme déjà indiqué, il existe manifestement un seuil de propriété entre les produits connus (à 2 - 3 %) et l'exemple de l'invention à 6 %.

Il est d'ailleurs tout à fait surprenant qu'un tel seuil brutal existe entre 3 et 6 %, qui n'était ni prévisible ni logique.

La fraction de polymères hydrosolubles pourra donc être de préférence entre 6 et 35 %, et encore de préférence entre 8 et 30 -35%.

QC1 : mesure de viscosité à pH=3

- Préparation d'une solution A à 1% en poids de matière active dans de l'eau déionisée puis ajustement du pH entre 2,9 et 3,1 en utilisant de l'acide chlorhydrique 1 N

- Mesure de la viscosité n°1 à l'aide d'un viscosimètre Brookfield de type RVT : les valeurs obtenues sont en centipoises

QC2: chute de viscosité par ajout de sels

- On ajoute à la solution A une solution saline (à 70 g/l de NaCl) afin d'obtenir une concentration finale en sel de 1 % massique par rapport à la matière active (polymère)

- Mesure de la viscosité n°2 à l'aide d'un viscosimètre Brookfield de type RVT : les valeurs obtenues sont en centipoises

- La chute de viscosité est calculée ainsi :

$$\text{Chute de viscosité (\%)} = 100 \times ((\text{viscosité 1} - \text{viscosité 2}) / \text{viscosité 1})$$

On peut constater que de manière surprenante les produits des exemples P1 à P6 présentent une bonne stabilité de leur viscosité en présence d'électrolytes, ce qui n'est pas le cas du produit X. Le taux de polymère hydrosoluble apparaît comme jouant un rôle primordial. Compte tenu de cette bonne résistance, les polymères de cette invention conservent une viscosité élevée même à pH extrêmement acide et ce contrairement aux produits connus sur le marché.

**Exemples de compositions :**

Dans le cadre des exemples de compositions, les deux polymères utilisés correspondent au polymère décrit précédemment sous l'appellation P1. Ce polymère a été choisi car il présente un bon compromis entre les valeurs QC1 et QC2. Toutefois, il est tout à fait envisageable d'utiliser également l'un ou l'autre des polymères présentés, cette sélection dépendant uniquement des caractéristiques finales requises pour la composition.

- Composant A : copolymère réticulé de type P1 comprenant 60% d'AMPS et 40% d'acide acrylique utilisé sous forme d'émulsion distillée contenant 62,6% de polymère

- Composant B : même polymère que le polymère A utilisé sous forme de poudre obtenue par « spray-drying »

Les noms des ingrédients utilisés dans les compositions sont ceux référencés sous la nomenclature INCI (« International Nomenclature of Cosmetic Ingredients»).

**Exemple 1 : base pour un gel / crème, pH 3,6 :**

|  |  | % en poids |
|---|---|---|
| Phase aqueuse : | glycérine | 2% |
|  | eau (aqua) | QSP 100 |
|  | acide citrique | QS pH = 3,5 |
| Composant A |  | 3% |
| conservateurs |  | Q S |
| parfum |  | Q S |
| acide citrique |  | QS pH final = 3,6 |

Mode opératoire :

1ère étape : préparation de la phase aqueuse.

2ème étape : ajustement du pH de la phase aqueuse par ajout d'acide.

3ème étape : on ajoute le composant A sous agitation.

4ème étape : on ajoute les conservateurs et le parfum.

5ème étape : réajustement du pH à 3,6

Caractéristiques finales de la composition :

- pH = 3,6

- viscosité (RVT 6, 20 tours/min) = 10250 cP = 10250 mPa.s

- aspect : gel / crème blanc, opaque et brillant

- toucher : onctueux car ce gel/crème se casse très progressivement à la surface de la peau. Texture : légère, non grasse et non collante

- préhension facile avec la main alors que la texture est relativement fluide

**Exemple 2 : lotion à base de silicone, pH 6:**

|  | % en poids |
|---|---|
| dimethicone | 10 % |
| palmitate d'octyle | 5% |
| eau (aqua) | QSP 100 |
| Composant A | 1,5 % |
| conservateurs | QS |
| parfum | QS |

Mode opératoire :

On incorpore les ingrédients dans l'ordre indiqué. On ajoute ensuite sous agitation le composant A, les conservateurs puis le parfum.

Caractéristiques finales de la composition :

- pH = 5,8

- viscosité (RVT 6, 20 tours/min) = 9500 cP = 9500 mPa.s

- aspect : crème brillante, facile à prendre à la main

- toucher : onctueux et léger, ne fait pas l' « eau » à l'application. Toucher final velouté

**Exemple 3 : base crème capillaire, pH 5 :**

|  | % en poids |
|---|---|
| eau (aqua) | QSP 100 |
| Huile d'olive (Olea Europaea) | 2 % |
| Composant B | 1,25 % |
| conservateurs | Q S |
| parfum | Q S |
| acide citrique | QS pH = 5 |

Mode opératoire :

On verse le composant B dans l'eau. On ajoute ensuite l'huile sous agitation. Dès que la préparation est homogène, on ajoute les conservateurs et le parfum. Le pH est ajusté à 5.

Caractéristiques finales de la composition :

- pH=5

- viscosité (RVT 6, 20 tours/min.) = 11500 cP = 11500 mPa.s

**Exemple 4 : base émulsion huile / eau démaquillante, pH 4,7 :**

|  |  |  | % en poids |
|---|---|---|---|
| Phase aqueuse : | Disodium Laureth sulfosuccinate | | 4% (m.a.) |
| | glycérine | | 3 % |
| | eau (aqua) | | QSP 100 |
| (m.a. = matières actives) | | | |
| Phase huileuse : | | | |
| | triglycéride caprylique/caprique | | 6 % |
| | Huile d'amande douce (Prunus Amigdalus Dulcis) | | |
| | | | 2% |
| Composant A | | | 4 % |
| Conservateurs | | | Q S |
| Parfum | | | Q S |

Mode opératoire :

On prépare la phase aqueuse La phase huileuse est ensuite incorporé. On ajoute alors sous agitation le composant A, puis les conservateurs et le parfum.

Caractéristiques finales de la composition :

- pH = 4,7

- viscosité (RVT 6, 20 tours/min.) = 4000 cP = 4000 mPa.s

- aspect : lait blanc

- toucher : mousse peu, facile à rincer, facile à étaler tout en gardant une texture consistante non grasse et non collante.

**Exemple 5 : base émulsion huile / eau, pH 4,2 :**

|  |  |  | % en poids |
|---|---|---|---|
| Phase huileuse | « Shea Butter » (Butyrospermum Parkii) **(beurre de karité)** | | 2 % |
| | Stéarate d'octyle | | 8 % |
| | Huile minérale (Paraffinum Liquidum) | | 4 % |
| eau (aqua) | | | QSP 100 |
| Composant A | | | 2 % |
| conservateurs | | | QS |
| parfum | | | Q S |
| acide citrique | | | QS pH 4,2 |

Mode opératoire :

Préparation la phase huileuse à 50°C. On ajoute l'eau et on maintient la température à 50°C. Le composant A est jouté sous agitation.

Le mélange est ensuite ramené à température ambiante afin d'y ajouter les conservateurs et le parfum. Le pH est alors ajusté.

Caractéristiques finales de la composition :

- pH = 4,2

- viscosité (RVT 6, 20 trs/min.) = 2750 cP = 2750 mPa.s

- aspect : fluide blanc opaque

- toucher : léger et consistant, permet le massage sans formation de particules inconfortables pour le consommateur

**Exemple 6 : base pour un gel / crème, pH 10 :**

|  | % en poids |
|---|---|
| eau (aqua) | QSP 100 |
| Composant A | 3% |
| NaOH | QS pH = 10 |
| conservateurs | Q S |
| parfum | Q S |

Mode opératoire :

On verse le composant A dans l'eau. On ajuste le pH à 10. On ajoute alors sous agitation les conservateurs et le parfum.

Caractéristiques finales de la composition :

- pH=10

- viscosité (RVT 6, 20 tours/min.) = 12000 cP= 12000 mPa.s

- aspect : gel / crème blanc, opaque et brillant

- toucher : onctueux. Le gel est facile à étaler.

Les exemples ci-dessus montrent la diversité des compositions qui peuvent être ciblées pour l'utilisation de ce type de polymères à la fois pour leurs propriétés épaississantes et/ou émulsionnantes.

Les améliorations obtenues sont nombreuses :

Les polymères épaississants et/ou émulsionnants tels que définis peuvent être incorporés à toute température. Ils offrent de plus une grande souplesse quant à l'étape d'incorporation.

Ce sont des polymères épaississants et émulsionnants efficaces et donc d'excellents stabilisants pour les compositions contenant des silicones, des huiles végétales, des ingrédients sous forme saline ou contenant des sels, ou ayant un pH inférieur à 6.

Chacune des compositions finales testées présente des caractéristiques cosmétiques optimales telles que le toucher et la stabilité.

Les touchers résultant des différentes formulations sont onctueux : au moment de l'application, la texture de la composition de l'invention se « casse » moins rapidement ce qui permet d'éviter un toucher trop « aqueux » (qui ressemble à de l'eau), peu attractif pour le consommateur, notamment en terme d'efficacité et de confort de la composition.

Cette propriété permet également d'éviter que la composition appliquée à la surface de la peau, des cheveux, des ongles ou des poils ne s'écoule trop rapidement. Il est alors plus facile de contrôler l'application et l'étalement de la composition sur la surface à traiter.

Il est à noter que pour chacune des formules possibles, le choix de l'emballage est vaste : flacon-pompe, tube, pulvérisateur ou « spray », pot,

De plus l'utilisation d'un polymère sous forme de poudre est un choix supplémentaire. Il permet de bénéficier des propriétés du polymère tel que décrit précédemment tout en évitant la présence de la phase huileuse (solvant du polymère) dans la composition finale. Cette possibilité offre une diversité supplémentaire permettant de répondre à de nouvelles exigences techniques et/ou marketing.

## Revendications

1. Utilisation comme épaississant et/ ou émulsionnant pour milieux aqueux (en totalité ou en partie) de compositions cosmétiques, dermatologiques, pharmaceutiques, à usage humain ou vétérinaire, ou détergentes, d'au moins un polymère obtenu par polymérisation de:

   - 5 à 95 % molaire d'au moins un monomère possédant une fonction acide faible, et de
   - 5 à 95 % molaire d'au moins un monomère possédant une fonction acide fort et
   - **caractérisé en ce qu'**il présente une fraction de polymères hydrosolubles comprise entre 5 et 50 % en poids (par rapport au polymère total), de préférence entre 6 et 35 %, de préférence 8 et 30 -35%, la méthode de détermination de la fraction hydrosoluble étant la suivante :

     - la méthode est basée sur la séparation des microgels de polymère réticulé d'avec une solution de polymère, par centrifugation, et la teneur en polymère est déterminée avant et après par titration de colloides, sur la base de la précipitation stoechiométrique de particules colloidales chargées par titration par un polymère de charge opposée, en utilisant un indicateur visuel,

   et **en ce que**, au moment de la polymérisation, la neutralisation de la globalité des monomères possédant une fonction acide est partielle et comprise entre 5 et 95%.

2. Utilisation d'un polymère selon la revendication 1, **caractérisée en ce que** la polymérisation est effectuée en émulsion inverse eau dans huile.

3. Utilisation d'un polymère selon la revendication 1 ou 2, **caractérisée en ce que** les polymères sont obtenus de plus en présence d'un agent réticulant ou ramifiant et éventuellement d'un agent de transfert.

4. Utilisation d'un polymère selon l'une quelconque des revendications 1 à 3. **caractérisée on ce que** le taux de réticulation ou ramification est de 50 à 3000 ppm (en considérant le méthylènebisacrylamide ou MBA) par rapport au polymère ou une réticulation équivalente avec un agent réticulant d'efficacité différente.

5. Utilisation d'un polymère selon l'une quelconque des revendications 1 à 4 **caractérisée en ce que** la concentration de polymérisation est de 15 à 55% en poids.

6. Utilisation d'un polymère selon l'une quelconque des revendications 1 à 5 **caractérisée en ce que** la polymérisation est menée éventuellement en présence d'un agent de transfert.

7. Utilisation d'un polymère selon l'une quelconque des revendications 1 à 6, **caractérisée en ce que** ledit polymère est obtenu à partir de :

   - 10 à 80% molaire d'au moins un monomère possédant une fonction acide faible et de
   - 20 à 90% molaire d'au moins un monomère possédant une fonction acide fort
   - et éventuellement de plus en présence d'un agent réticulant ou ramifiant et éventuellement d'un agent de transfert.

8. Utilisation d'un polymère colon l'une quelconque des revendications 1 à 7 **caractérisée en ce que** le taux de neutralisation (au moment de la polymérisation) de l'ensemble des acides insaturés dudit polymère est de préférence de 10 à 80%

9. Utilisation d'un polymère selon l'une quelconque des revendications 1 à 8 **caractérisée en ce que** le taux de réticulation ou ramification dudit polymère est situé de préférence entre 100 et 800 ppm (en considérant le MBA) par rapport au polymère ou une réticulation équivalente avec un agent réticulant d'efficacité différente.

10. Utilisation d'un polymère selon l'une quelconque des revendications 1 à 9 **caractérisée en ce que**, dans les mêmes conditions, la polymère qui serait obtenu en l'absence d'agent de réticulation aurait une viscosité intrinsèque (I.V.) de 3 à 25 dl/g.

11. Utilisation selon l'une quelconque des revendications 1 à 1c' **caractérisée en ce que** la concentration de polymérisation est de préférence de 25 à 45% de matière active en poids.

12. Utilisation selon l'une quelconque des revendications 1 à 9 **caractérisée en ce que** le taux global de neutralisation des monomères possédant une fonction acide est compris entre 20 et 90% à a polymérisation

13. Utilisation selon l'une quelconque des revendications 1 à 12 **caractérisée en ce que** lors de la polymérisation sous forme d'émulsion eau dans huile, la phase continue Utilisée peut être une huile ou un solvant d'origine minéraie et/ou de synthèse et/ou d'origine végétale, de préférence, un solvant ou une huile non minérale.

14. Utilisation selon l'une quelconque des revendications 1 à 13 **caractérisée en ce que** ledit polymère comprend comme monomère run des monomètres de type acide fort suivants:

   - monomères présentant une fonction de type acide sulfonique, acide phosphonique, par exemple : l'acide 2-acrylamido-2-methylpropane sulfonique (AMPS).

15. Utilisation selon l'une quelconque des revendications 1 à 14 **caracterisée en ce que** ledit polymère comprend comme monomère l'un des monomères de type acide faible suivants:

   - l'acide acrlique, l'acide méthacrylique, l'acide itaconique, l'acide crotonique, l'acide maléique, l'acide fumarique.

16. Utilisation selon l'une quelconque des revendications 1 à 15, **caractérisée en ce que** ledit polymère a été réticulé (ou ramifié) par l'un des réticulants suivants: le méthylènebisacrylamide (MBA), l'ethylene glycol diacrylate le polyethylene glycol dimethacrylate, le diacrylamide, le cyanomethylacrylate, le vinyloxyethylacrylate ou methacrylate et le formaldenyde, le glyoxal, les composés de type glycidyléther comme l'éthylèriegiycol diglycidyléther, ou des époxy.

17. Utilisation selon l'une quelconque des revendications 1 à 16, **caractérisée en ce que** ledit polymère comprend l'un des agents de transfert ci-dessdus: alcool isopropylique, hypophosphite de sodium, mercaptbethanol.

18. Utilisation selon l'une quelconque des revendications 1 à 17, **caractérisée en ce que** ledit polymère est formé en émulsion inverse à partir de :

| | P1 | P2 | P3 | P4 | P5 | P6 |
|---|---|---|---|---|---|---|
| % molaire AMPS (acide fort) | 6 0 | 60 | 60 | 40 | 50 | 90 |
| % molaire Ac. Acrylique (acide faible) | 40 | 40 | 40 | 60 | 50 | 10 |
| % Neutralisation (total) | 7 0 | 50 | 36 | 55 | 60 | 70 |
| % Matière Active | 35 | 35 | 30 | 30 | 30 | 36 |
| % Polymère Hydrosoluble | 1 7 | 6 | 15 | 10 | 28 | 20 |
| OC1 (mesure de viscosité à pH=3) | 6000 | 16500 | 12000 | 3250 | 4500 | 12500 |
| OC2 (chute de viscocité par ajout de sole) | 54 | 70 | 43 | 54 | 44 | 60 |

**Claims**

1. Use as a (total or partial) thickener and / or emulsifier for aqueous media in cosmetic and dermatological compositions, pharmaceutical compositions for human or veterinary use, or detergents, of at least one polymer obtained through polymerisation of:

   - 5 to 95 mole % of at least one monomer having a weak acid function, and of
   - 5 to 95 mole % of at least one monomer having a strong acid function and
   - wherein the polymer contains a fraction of hydrosoluble polymers of between 5 and 50% by weight (of the total polymer), preferably between 6 and 35%, preferably 8 and 30-35%, the method for determining the hydrosoluble fraction being the following:
   - the method is based on the separation of the cross-linked polymer microgels with a polymer solution, by centrifuging, and the polymer content is determined before and after by colloid titration, on the basis of the stoichiometric precipitation of charged colloidal particles through titration of a polymer of the opposite charge, using a visual indicator, and wherein, at the moment of polymerisation, the neutralisation of the totality of the monomers having an acid function is partial and between 5 and 95%.

2. Use of a polymer according to claim 1, wherein the polymerisation is carried out in an inverse water-in-oil emulsion.

3. Use of a polymer according to claims 1 and 2, wherein more polymers are obtained in the presence of a cross-linking or branching agent, or possibly a transfer agent.

4. Use of a polymer according to any of claims 1 to 3, wherein the rate of cross-linking or branching is between 50 and 3000 ppm (considering the methylene-bis-acrylamide or MBA) in the polymer, or an equivalent level of cross-linking with a cross-linking agent with a different effectiveness.

5. Use of a polymer according to any of claims 1 to 4, wherein the polymerisation concentration is between 15 and 55% by weight.

6. Use of a polymer according to any of claims 1 to 5, wherein the polymerisation is possibly controlled by the presence of a transfer agent.

7. Use of a polymer according to any of claims 1 to 6, wherein said polymer is obtained from:

   - 10 to 80 mole % of at least one monomer having a weak acid function and from
   - 20 to 90 mole % of at least one monomer having a strong acid function
   - and possibly more in the presence of a cross-linking or branching agent and possibly a transfer agent.

8. Use of a polymer according to any of claims 1 to 7, wherein the rate of neutralisation (at the moment of polymerisation) of all the unsaturated acids of said polymer is preferably between 10 and 80%.

9. Use of a polymer according to any of claims 1 to 8, wherein the cross-linking or branching rate of said polymer is preferably between 100 and 800 ppm (taking into account the MBA) in relation to the polymer or an equivalent level of cross-linking with a cross-linking agent with a different efficiency.

10. Use of a polymer according to any of claims 1 to 9, wherein under the same conditions the polymer obtained without a cross-linking agent has an intrinsic viscosity (IV) of 3 to 25 dl/g.

11. Use according to any of claims 1 to 10, wherein the polymerisation concentration is preferably 25 to 45% by weight of the active material.

12. Use according to any of claims 1 to 9, wherein the overall neutralisation rate of monomers having an acid function is between 20 and 90% during polymerisation.

13. Use according to any of claims 1 to 12, wherein during polymerisation in the form of water-in-oil emulsion, the continuous phase used may be an oil or solvent of mineral and/or synthetic and/or vegetal origin, preferably a non-mineral solvent or oil.

14. Use according to any of claims 1 to 13, wherein said polymer comprises as a monomer one of the following strong acid monomers:

   - monomers presenting a sulfonic acid function or a phosphonic acid function, for example: 2-acrylamido-2-methylpropane sulfonic acid (AMPS).

15. Use according to any of claims 1 to 14, wherein said polymer comprises as a monomer one of the following weak acid monomers:

   - acrylic acid, methacrylic acid, itaconic acid, crotonic acid, maleic acid, fumaric acid.

16. Use according to any of claims 1 to 15, wherein said polymer has been cross-linked (or branched) by one of the following cross-linking agents: methylene-bis-acrylamide (MBA), ethylene glycol diacrylate, polyethylene glycol dimethacrylate, diacrylamide, cyano methyl acrylate, vinyloxy ethyl acrylate or methacrylate and formaldehyde, glyoxal, glycidyl ether type components such as ethylene glycol diglycidyl ether, or epoxies.

17. Use according to any of claims 1 to 16, wherein said polymer comprises one of the following transfer agents: isopropylic alcohol, sodium hypophosphite, mercaptoethanol.

18. Use according to any of claims 1 to 17, wherein said polymer is formed by means of inverse emulsion polymerisation using:

|  | P1 | P2 | P3 | P4 | P5 | P6 |
|---|---|---|---|---|---|---|
| % mole AMPS (strong acid) | 60 | 60 | 60 | 40 | 50 | 90 |
| % mole acrylic acid (weak acid) | 40 | 40 | 40 | 60 | 50 | 10 |
| % (Total) Neutralisation | 70 | 50 | 36 | 55 | 60 | 70 |
| % Active Material | 35 | 35 | 30 | 30 | 30 | 35 |
| % Hydrosoluble Polymer | 17 | 6 | 15 | 10 | 28 | 20 |
| QC1 (viscosity measurement at pH=3) | 6000 | 16500 | 12000 | 3250 | 4500 | 12500 |
| QC2 (fall in viscosity by adding salts) | 54 | 70 | 43 | 54 | 44 | 60 |

**Patentansprüche**

1. Verwendung als Verdickungsmittel und / oder Emulgator in wässriger Lösung (ganz oder teilweise) in Zusammensetzungen der Kosmetik, Dermatologie, Pharmazie, zur humanen- oder veterinären Verwendung, oder als Detergenzien von wenigstens einem Polymer, erhalten durch die Polymerisation von:

   - 5 bis 95 Mol-% wenigstens eines Monomers mit einer schwachen Säurefunktion und
   - 5 bis 95 Mol-% wenigstens eines Monomers mit einer starken Säurefunktion und
   - **dadurch gekennzeichnet, dass** eine Fraktion wasserlöslicher Polymere mit 5 bis 50 Gew.-% vorliegt (bezogen auf das Gesamtpolymer), bevorzugt zwischen 6 bis 35 %, vorzugsweise zwischen 8 und 30 - 35 %, wobei das Verfahren der Bestimmung der wasserlöslichen Fraktion das folgende ist:
   - das Verfahren basiert auf der Trennung von Mikrogelen des vernetzten Polymers mit einer Polymerlösung, durch Zentrifugation, und der Bestimmung des Polymergehaltes vor und nach der Titration von Kolloiden, aufgrund der stöchiometrischen Fällung der geladenen kolloidalen Partikel durch Titration mit einem entgegengesetzt geladenen Polymer und der Verwendung eines sichtbaren Indikators und dass, zum Zeitpunkt der Polymerisation, die Neutralisation des gesamten Monomers mit einer sauren Funktion teilweise vorliegt, und zwischen 5 bis 95 % ist.

2. Verwendung eines Polymers nach Anspruch 1, **dadurch gekennzeichnet dass** die Polymerisation in einer umgekehrten Wasser-in-Öl-Emulsion durchgeführt wird.

3. Verwendung eines Polymers nach Anspruch 1 oder 2, **dadurch gekennzeichnet dass** die Polymere zusätzlich in der Anwesenheit eines vernetzenden Agens oder eines verzweigten Agens oder gegebenenfalls eines Übergangsagens erhalten wurden.

4. Verwendung eines Polymers nach einem der Ansprüche 1 bis 3, **dadurch gekennzeichnet dass** der Grad der Vernetzung oder Verzweigung von 50 bis 3000 ppm ist (unter Berücksichtigung des Methylenbisacrylamids oder MBA) im Verhältnis zum Polymer oder eines vernetzenden Äquivalentes mit einem vernetzenden Agens anderer Wirkung.

5. Verwendung eines Polymers nach einem der Ansprüche 1 bis 4, **dadurch gekennzeichnet dass** die Polymerkonzentration zwischen 15 bis 55 Gew.-% ist.

6. Verwendung eines Polymers nach einem der Ansprüche 1 bis 5, **dadurch gekennzeichnet dass** das die Polymerisation gegebenenfalls in der Anwesenheit eines Übergangsagens durchgeführt wird.

7. Verwendung eines Polymers nach einem der Ansprüche 1 bis 6, **dadurch gekennzeichnet dass** das besagte Polymer erhalten wird durch:

   - 10 bis 80 Mol-% wenigstens eines Monomers mit einer schwachen Säurefunktion und
   - 20 bis 90 Mol-% wenigstens eines Monomers mit einer starken Säurefunktion
   - und gegebenenfalls zusätzlich in der Anwesenheit eines vernetzenden Agens oder eines verzweigten Agens oder gegebenenfalls eines Übergangsagens.

8. Verwendung eines Polymers nach einem der Ansprüche 1 bis 7, **dadurch gekennzeichnet dass** der Grad der Neutralisation (zum Zeitpunkt der Polymerisation) der ungesättigten Säuren des besagten Polymers vorzugsweise zwischen 10 bis 80 % ist.

9. Verwendung eines Polymers nach einem der Ansprüche 1 bis 8, **dadurch gekennzeichnet dass** der Grad der Vernetzung oder Verzweigung des besagten Polymers vorzugsweise zwischen 100 bis 800 ppm liegt (unter Berücksichtigung des MBA) im Verhältnis zum Polymer oder eines vernetzenden Äquivalentes mit einem vernetzenden Agens anderer Wirkung.

10. Verwendung eines Polymers nach einem der Ansprüche 1 bis 9, **dadurch gekennzeichnet dass** unter den gleichen Bedingungen, das Polymer, welches bei Abwesenheit von einem vernetzenden Agens erhalten würde, eigentlich eine Viskosität (1.V.) von 3 bis 25 dl/g hätte.

11. Verwendung nach einem der Ansprüche 1 bis 10, **dadurch gekennzeichnet dass** der Polymerkonzentration bevorzugt zwischen 25 bis 45 Gew.-% des aktivierenden Stoffes ist.

12. Verwendung nach einem der Ansprüche 1 bis 9, **dadurch gekennzeichnet dass** der gesamte Grad der Neutralisation der Monomere mit einer saueren Funktion zwischen 20 bis 90 % der Polymerisation liegt.

13. Verwendung eines Polymers nach einem der Ansprüche 1 bis 12, **dadurch gekennzeichnet dass** bei der Polymerisation unter Bildung einer Öl-in-Wasser-Emulsion, als Dispersionsmittel ein Öl oder eine Solvens mineralischen und/oder synthetischen und/oder pflanzlichen Ursprungs, vorzugsweise ein nichtmineralisches Solvens oder Öl, verwendet wird.

14. Verwendung eines Polymers nach einem der Ansprüche 1 bis 13, **dadurch gekennzeichnet dass** das besagte Polymer als Monomer eines der Monomere sauren Typs der folgenden umfasst:

   - Monomere mit einer Funktion des Typs Schwefelsäure, Phosphorsäure, zum Beispiel: 2-Acrylamid-2-Methylpropan Schwefelsäure (AMPS)

15. Verwendung eines Polymers nach einem der Ansprüche 1 bis 14, **dadurch gekennzeichnet dass** das besagte Polymer als Monomer eines der Monomere des schwach sauren Typs der folgenden umfasset:

   - Acrylsäure, Methacrylsäure, Itakonsäure, Krotonsäure, Maleinsäure, Fumarsäure.

**16.** Verwendung nach einem der Ansprüche 1 bis 15, **dadurch gekennzeichnet dass** das besagte Polymer vernetzt ist (oder verzweigt) durch einen der folgenden Vernetzer:

Methylenbisacrylamid (MBA), Ethylenglycoldiacrylat, Polyethylenglycoldimethylacrylat, Diacrylamid, Cyanmethylacrylat, Vinyloxyethylacrylat oder Methacrylat und Formaldehyd, Glyoxal, Verbindungen des Typs Glyzidylether wie Ethylenglycol, Diglyzidylether, oder Epoxy.

**17.** Verwendung eines Polymers nach einem der Ansprüche 1 bis 16, **dadurch gekennzeichnet dass** das besagte Polymer eines der folgenden Übergangsagenzien enthält: Isopropylalkohol, Natriumhydrophosphit, Mercaptoethanol.

**18.** Verwendung eines Polymers nach einem de Ansprüche 1 bis 17, **dadurch gekennzeichnet dass** das besagtes Polymer in einer umgekehrten Emulsion gebildet wird aus:

|  | P1 | P2 | P3 | P4 | P5 | P6 |
|---|---|---|---|---|---|---|
| % mol. AMPS (starke Säure) | 60 | 60 | 60 | 40 | 50 | 90 |
| % mol AC, Acryl (schwache Säure) | 40 | 40 | 40 | 60 | 50 | 10 |
| % Neutralisation (gesamt) | 70 | 50 | 36 | 55 | 60 | 70 |
| % aktivierender Stoff | 35 | 35 | 30 | 30 | 30 | 36 |
| % wasserlösliches Polymer | 17 | 6 | 15 | 10 | 28 | 20 |
| OC1 (Messung der Viskosität bei pH = 3) | 6000 | 16500 | 12000 | 3250 | 4500 | 12500 |
| OC2 (Abfall der Viskosität durch Zusatz von Salzen) | 54 | 70 | 43 | 54 | 44 | 60 |

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2416723
- EP 0161038 A
- GB 2077750 A
- US 4172066 A
- EP 0503853 A
- GB 2110744 A
- WO 9942521 A
- WO 9936445 A
- EP 0186361 A
- EP 0343840 A